# EUROPEAN PATENT APPLICATION

(11) **EP 4 506 013 A1**
(43) Date of publication of application: **12.02.2025**
(21) Application number: 22934308.2
(22) Date of filing: 01.04.2022
(51) Int. Cl.: A61K 35/36, A61K 39/285, A61P 25/16

(54) **USE OF EXTRACT FROM RABBIT SKIN INFLAMED BY VACCINIA VIRUS IN TREATING PARKINSON'S DISEASE**

(71) Applicant: Star Bright Bio-Tech Limited, Kowloon Hong Kong (HK)
(72) Inventor: LAU, Shing Hing, Hong Kong (CN)
(74) Representative: Rüger Abel Patentanwälte PartGmbB
(86) International application number: PCT/CN2022/084780
(87) International publication number: WO 2023/184471

(57) **Abstract**

The use of extract from rabbit skin inflamed by vaccinia virus for treating Parkinson's disease or restoring neurological function of the brain or alleviating damage to neurological function of the brain in a patient suffering from Parkinson's disease is provided. The extract from rabbit skin inflamed by vaccinia virus can be Lepalvir.

## Description

### Field of invention

The invention relates to the field of medicine. Specifically, the present invention relates to new therapeutic use of extract from rabbit skin inflamed by vaccinia virus. More specifically, the present invention relates to a medical use of extract from rabbit skin inflamed by vaccinia virus for treating Parkinson's disease.

### Background Art

Parkinson's disease usually occurs in people over 60 years old, and about 1% of the elderly worldwide suffer from the disease. Symptoms of Parkinson's disease usually appear slowly over time. The most obvious early symptoms are tremors, limb stiffness, motor impairment and abnormal gait. There may also be cognitive and behavioral problems; dementia is quite common in patients with severe illness, and more than one-third of cases also develop major depression and anxiety. The apoptosis of dopaminergic neurons in the substantia nigra pars compacta can lead to Parkinson's disease.

Parkinson's disease may be hereditary or may be caused by some unknown factors. Parkinson's disease may also be caused by viral infection or toxins such as MPTP (1-methyl-4-phenyl-1,2,3,6-tetrahydropyridine). MPTP can be used as a drug to induce Parkinson 's disease in experimental animals (Langston, J. W et al., Chronic Parkinsonism in humans due to a product of meperidine-analog synthesis . Science (New York, NY). 1983-02-25, 219 (4587): 979-980).

Unlike stroke or other acute cerebrovascular diseases, Parkinson's disease is a chronic disease and is not caused by acute cerebral hypoxia and toxicity due to cerebral vascular embolism or rupture.

In terms of pathophysiology, Parkinson's disease is considered a synucleinopathy due to the accumulation of α-synuclein in the form of Lewy bodies (Schulz-Schaeffer, Walter J. The synaptic pathology of α-synuclein aggregation in dementia with Lewy bodies, Parkinson's disease and Parkinson's disease dementia . Acta Neuropathologica. 2010-8, 120 (2): 131-143). This is very different from the neurofibrillary tangles formed by the accumulation of Tau proteins in Alzheimer's disease. The main pathological changes in Parkinson's disease occur in the compact part of the ventral substantia nigra in the midbrain. This area contains a large number of dopaminergic neurons and signals to the basal ganglia of the brain (Obeso, Jose A et al., Functional organization of the basal ganglia: therapeutic implications for Parkinson's disease . Movement Disorders: Official Journal of the Movement Disorder Society. 2008,. 23 Suppl 3: S548-559). A patient suffering from Parkinson's disease have a large number of neuronal deaths in the pars compacta, and some even lose up to 70% of their neurons (Davie, CA, A review of Parkinson's disease. British Medical Bulletin. 2008, 86: 109-127).

Selegiline is a monoamine oxidase (MAO)-B inhibitor (MAOI). In 2006, Selegiline was approved by the US FDA for the treatment of Parkinson's disease.

There is a need in the art for drugs that are effective in treating Parkinson's disease .

The extract from rabbit skin inflamed by vaccinia virus refers to active substances extracted from the rabbit skin that has been inflamed after being vaccinated with vaccinia virus. This extract from rabbit skin inflamed by vaccinia virus is commercially available under the trade name Lepalvir for pain treatment. The extract has been shown to be clinically safe and has no obvious side effects. In addition, WO2020/211009 discloses the use of extract from rabbit skin inflamed by vaccinia virus for the treatment of hematopoietic system damage; WO 2020/248240 discloses the use of extract from rabbit skin inflamed by vaccinia virus for the treatment of cancer, and their entire contents are incorporated herein by reference.

### Summary of the invention

The purpose of the present invention includes providing a drug for preventing, alleviating or treating Parkinson's disease. The technical problem of the present invention is solved by providing extract from rabbit skin inflamed by vaccinia virus, preferably Lepalvir.

In general, the inventors have found that extract from rabbit skin inflamed by vaccinia virus can effectively prevent, treat or alleviate Parkinson's disease. In addition, the inventors unexpectedly found that the effect of extract from rabbit skin inflamed by vaccinia virus in treating the disease is comparable to that of the approved drug Selegiline.

In one aspect, the present invention relates to the use of extract from rabbit skin inflamed by vaccinia virus in the preparation of a medicament for preventing or treating Parkinson's disease in a patient. In one aspect, the present invention relates to extract from rabbit skin inflamed by vaccinia virus for use in preventing or treating Parkinson's disease in a patient. In one aspect, the present invention relates to a method for preventing or treating Parkinson's disease in a patient in need thereof, the method comprising administering a therapeutically effective amount of extract from rabbit skin inflamed by vaccinia virus to the patient.

In one aspect, the extract from rabbit skin inflamed by vaccinia virus or the medicament is used to restore neurological function of the brain or alleviate damage to neurological function of the brain in a patient suffering from Parkinson's disease. Damage to neurological function of the brain can be caused by degeneration, damage, reduction or death of brain neurons, especially degeneration, reduction, damage or death of neurons in the substantia nigra pars compacta of the brain.

The neurons described herein may be dopaminergic neurons. Dopaminergic neurons refer to neurons that contain and release dopamine as a neurotransmitter.

In one aspect, the present invention relates to the use of extract from rabbit skin inflamed by vaccinia virus in the preparation of a medicament for alleviating or reducing neuronal degeneration, damage or death in the brain of a patient suffering from Parkinson's disease. In one aspect, the present invention relates to extract from rabbit skin inflamed by vaccinia virus for use in alleviating or reducing neuronal degeneration, damage or death in the brain of a patient suffering from Parkinson's disease. In one aspect, the present invention relates to a method for alleviating or reducing neuronal degeneration, damage or death in the brain of a patient suffering from Parkinson's disease, the method comprising administering a therapeutically effective amount of extract from rabbit skin inflamed by vaccinia virus to the patient. The neurons may be neurons in the substantia nigra pars compacta, preferably dopaminergic neurons.

In one aspect, Parkinson's disease is treated by alleviating or reducing neuronal degeneration, damage or death in the brain of the patient.

In one aspect, the patient suffers from Parkinsonism caused by Parkinson's disease. Therefore, the present invention also relates to the use of extract from rabbit skin inflamed by vaccinia virus in the preparation of a medicament for preventing or treating Parkinsonism in a patient. In one aspect, the present invention relates to extract from rabbit skin inflamed by vaccinia virus for use in preventing or treating Parkinsonism in a patient. In one aspect, the present invention relates to a method for preventing or treating Parkinsonism in a patient in need thereof, the method comprising administering a therapeutically effective amount of extract from rabbit skin inflamed by vaccinia virus to the patient.

In one aspect, the patient with Parkinson's disease or Parkinsonism has motor symptoms such as tremor, limb rigidity, bradykinesia or postural instability; neuropsychiatric disorder such as speech disorder, cognitive disorder, emotional disorder or thought disorder; sensory disorder or sleep disorder; or autonomic dysfunction such as excessive oil secretion, hyperhidrosis or urinary incontinence. The present invention relates to use of the extract in alleviating these symptoms in the patient.

Parkinson's disease can be caused by aging, genetic factors, environmental toxins or pathogen infection.

Parkinson's disease or Parkinsonism is characterized by a decrease, damage or death of neurons in the substantia nigra pars compacta of the brain.

In one aspect, the extract from rabbit skin inflamed by vaccinia virus can be used as the only active ingredient for the treatment of Parkinson's disease or Parkinsonism. In this aspect, extract from rabbit skin inflamed by vaccinia virus can be used as the only active ingredient for the preparation of a medicament for the treatment of Parkinson's disease or Parkinsonism, or for restoring neurological function of the brain or alleviating damage to neurological function of the brain in a patient suffering from Parkinson's disease, or for alleviating or reducing neuronal degeneration, damage or death in the brain of a patient suffering from Parkinson's disease. In the treatment method of the present invention, the extract from rabbit skin inflamed by vaccinia virus can be administered to the patient as the only active ingredient. "The only active ingredient" means that the extract from rabbit skin inflamed by vaccinia virus is not used, administered to a patient, or prepared as a medicament, in combination with other therapeutic agents for Parkinson's disease. In one aspect, the present invention relates to a pharmaceutical composition comprising extract from rabbit skin inflamed by vaccinia virus and optionally a pharmaceutically acceptable carrier, adjuvant or excipient. In one aspect of the present invention, the pharmaceutically acceptable carrier, adjuvant or excipient are those that formulate the drug into an oral formulation or an injection. In one aspect, the extract from rabbit skin inflamed by vaccinia virus is formulated into an oral formulation or an injection, preferably an intramuscular injection or an intravenous injection. In one aspect, the extract from rabbit skin inflamed by vaccinia virus is Lepalvir. Accordingly, the present invention also relates to the pharmaceutical composition for the treatment of Parkinson's disease, or for restoring neurological function of the brain or alleviating damage to neurological function of the brain in a patient suffering from Parkinson's disease, or for alleviating or reducing neuronal degeneration, damage or death in the brain of a patient suffering from Parkinson's disease. The present invention also relates to the use of the extract from rabbit skin inflamed by vaccinia virus in the preparation of the pharmaceutical composition.

In one aspect, the extract from rabbit skin inflamed by vaccinia virus is Lepalvir.

In one aspect, the extract from rabbit skin inflamed by vaccinia virus is formulated into an oral preparation or an injection, preferably an intramuscular injection or an intravenous injection.

In one aspect, the patient is a mammal, preferably a human.

In one aspect, the extract from rabbit skin inflamed by vaccinia virus is administered to a patient, such as a human, in an amount of 0.05 U/kg to 50 U/kg, preferably 0.1 U/kg to 10 U/kg, more preferably 0.5 U/kg to 5 U/kg.

In one aspect, the prepared medicament comprises 3 U to 3000 U, preferably 6 U to 600 U, more preferably 30 U to 300 U of the extract from rabbit skin inflamed by vaccinia virus.

In one aspect, the extract from rabbit skin inflamed by vaccinia virus (preferably Lepalvir) is administered to a patient, preferably a human, in an amount of 0.05 U/kg to 50 U/kg, preferably 0.1 U/kg to 10 U/kg, more preferably 0.5 U/kg to 5 U/kg. For example, the extract from rabbit skin inflamed by vaccinia virus is administered to a patient, preferably a human, in an amount selected from the group consisting of: 0.05 U/kg, 0.06 U/kg, 0.07 U/kg, 0.08 U/kg, 0.09 U/kg, 0.1 U/kg, 0.2 U/kg, 0.3 U/kg, 0.4 U/kg, 0.5 U/kg, 0.6 U/kg, 0.7 U/kg, 0.8 U/kg, 0.9 U/kg, 1 U/kg, 1.5 U/kg, 2 U/kg, 2.5 U/kg, 3 U/kg, 3.1 U/kg, 3.2 U/kg, 3.3 U/kg, 3.4 U/kg, 3.5 U/kg, 3.6 U/kg, 3.7 U/kg, 3.8 U/kg, 3.9 U/kg, 4 U/kg, 4.5 U/kg, 5 U/kg, 5.5 U/kg, 6 U/kg, 6.5 U/kg, 7 U/kg, 7.5 U/kg, 8 U/kg, 8.5 U/kg, 9 U/kg, 9.5 U/kg, 10 U/kg, 11 U/kg, 12 U/kg, 13 U/kg, 14 U/kg, 15 U/kg, 16 U/kg, 17 U/kg, 18 U/kg, 19 U/kg, 20 U/kg, 25 U/kg, 30 U/kg, 35 U/kg, 40 U/kg, 45 U/kg, 50 U/kg and the ranges bounded by these numbers. It is known to those skilled in the art that for the dosage, human dose (U/kg or mg/kg) = mouse dose (U/kg or mg/kg) / 12.3; or human dose (U/kg or mg/kg) = mouse dose (U/kg or mg/kg) × 0.08. The above dosage can be an effective amount for treating the above-mentioned diseases in a patient. In one aspect, the extract from rabbit skin inflamed by vaccinia virus is administered by injection at the above-mentioned dose, such as intramuscular injection or intravenous injection.

In one aspect of the present invention, the prepared medicament or pharmaceutical composition comprises 3 U to 3000 U, preferably 6 U to 600 U, more preferably 30 U to 300 U of the extract from rabbit skin inflamed by vaccinia virus. The medicament or pharmaceutical composition is used to administer to a human, such as an adult. The average weight of an adult is, for example, 60 kg. Accordingly, the amount of extract from rabbit skin inflamed by vaccinia virus contained in the medicament prepared by the present invention is, for example, 3 U, 4 U, 5 U, 6 U, 7 U, 8 U, 9 U, 10 U, 15 U, 20 U, 25 U, 30 U, 35 U, 40 U, 42 U, 44 U, 45 U, 46 U, 47 U, 48 U, 49 U, 50 U, 55 U, 60 U, 65 U, 70 U, 80 U, 90 U, 100 U, 120 U, 150 U, 160 U, 170 U, 180 U, 190 U, 192 U, 194 U, 195 U, 196 U, 198 U, 200 U, 220 U, 240 U, 260 U, 280 U, 300 U, 350 U, 400 U, 500 U, 600 U, 700 U, 800 U, 900 U, 1000 U, 1500 U, 2000 U, 2500 U, 3000 U and the ranges bounded by these numbers. In one aspect, the medicament or pharmaceutical composition is prepared as an injection, such as an intramuscular injection or an intravenous injection. In one aspect, the medicament or injection is a fixed dose that cannot be divided. In one aspect, the medicament or injection cannot be divided into smaller doses within 1, 2, 3, 4, 5, 6 or 7 days. In one aspect, the medicament or injection is administered only once within 1, 2, 3, 4, 5, 6 or 7 days.

In one aspect, the extract from rabbit skin inflamed by vaccinia virus is administered to a patient every 6-72 hours, preferably 12-48 hours, more preferably 24-36 hours, more preferably 24 hours.

In one aspect, the regimen of administration to the patient is three times a day, twice a day, once a day, once every two days, once every three days, once every four days, once every five days, once every six days, once a week, once every two weeks, once every three weeks, once a month. For example, the extract of the present invention can be administered to the patient once a day.

In one aspect, the extract of the present invention is administered to the patient for at least 24 months, at least 12 months, at least 6 months, at least 2 months, at least 1 month, at least 3 weeks, at least 2 weeks, at least 10 days, at least 7 days, at least 5 days, at least 2 days, or at least 1 day.

As used herein, "extract from rabbit skin inflamed by vaccinia virus" refers to an extract, which contains active substances and is extracted from the inflamed rabbit skin inoculated with vaccinia virus using such processes as leaching, purification, and refining. This extract is usually a yellow or light yellow liquid, but it can also be dried into a solid. The injection of this extract from rabbit skin inflamed by vaccinia virus is commercially available under the trade name Lepalvir.

In the preparation of the extract of the present invention, the preparation method comprises extracting rabbit skin fragments inflamed after vaccinia virus inoculation with a phenol aqueous solution, wherein preferably a phenol aqueous solution with a phenol concentration of about 1%-10%, preferably about 2%-5%, more preferably about 2% or about 3% is used at a temperature below about 12°C, such as about 0-10°C, preferably about 2-8°C, more preferably about 3-6°C, and more preferably about 4°C. In addition, the preparation method further comprises adsorbing the extract treated with the phenol aqueous solution with an adsorbent (such as activated carbon), and desorbing under alkaline conditions, wherein preferably the adsorption is carried out under acidic conditions (such as about pH 3-6, more preferably about pH 4-5, more preferably about pH 4.5), and the alkaline conditions for desorption are about pH 9-12, preferably about pH 10 or pH 11.

In one aspect, the extract from rabbit skin inflamed by vaccinia virus or Lepalvir can be prepared by a method including the following steps:
(1) The inflamed rabbit skin after inoculation with vaccinia virus is collected, fragmented, and extracted with an extraction solvent to obtain solution A;
(2) Solution A is treated with acid and heat to obtain solution B;
(3) Solution B is treated with alkali and heat to obtain solution C;
(4) Solution C is subject to adsorption and filtration under acidic conditions, and desorption under alkaline conditions to obtain solution D;
(5) Solution D is neutralized and heated to obtain solution E;
(6) Solution E is concentrated to obtain the extract; and
(7) Optionally the extract is mixed with pharmaceutically acceptable carrier, adjuvant or excipient.

In one aspect, in step (1), the rabbit is inoculated with vaccinia virus; the skin with pox is collected; the skin is fragmented; an aqueous solution of phenol is added, and the skin is soaked at a temperature of lower than about 12°C (for example, about 0-10°C, preferably about 2-8°C, more preferably about 3-6°C, more preferably about 4°C) for at least about 12 hours (for example, about 24-90 hours, preferably about 48-72 hours, more preferably about 70 or about 72 hours). The supernatant is obtained by centrifugation, and solution A is obtained by filtration. The phenol concentration in the phenol aqueous solution is about 1%-10%, preferably about 2%-5%, more preferably about 2% or about 3%.

In one aspect, in step (2), the solution A is adjusted to acidic (for example, about pH 4-6, more preferably about pH 4.5-5.5, more preferably about pH 5) with acid (for example, hydrochloric acid), and heated (for example, at about 90-100°C, preferably about 95°C for at least about 10 minutes, such as about 20-50 minutes, preferably about 30-40 minutes), optionally lowering the temperature (for example, to less than about 50°C, preferably less than about 30°C), followed by centrifugation to obtain the supernatant, and filtration to obtain solution B. The step (2) can be performed in a nitrogen environment.

In one aspect, in step (3), the solution B is adjusted to alkaline (e.g., about pH 8-10, more preferably about pH 8.5-9.5, more preferably about pH 9 or about pH 9. 2), and heated (for example, at about 90-100°C, preferably about 95°C for at least 10 minutes, such as about 30-50 minutes, preferably about 30-40 minutes), optionally lowering the temperature (for example, to less than about 50°C, preferably less than about 30°C), followed by filtration to obtain solution C. The step (3) can be performed in a nitrogen environment.

In one aspect, in step (4), the solution C is adjusted to acidic (for example, about pH 3-6, more preferably about pH 4-5, more preferably about pH 4.5) with an acid (for example, hydrochloric acid), and an adsorbent is added thereto (For example, activated carbon) for soaking (for example, under stirring for at least about 1 hour, preferably about 2-10 hours, more preferably about 4 hours), after which the solution is removed and the adsorbent containing the active ingredient is collected. Subsequently, the above-mentioned adsorbent is added to the eluent (for example, water), and the pH is adjusted to alkaline (for example, about pH 9-12, preferably about pH 10 or pH 11) with a base (for example, sodium hydroxide) to separate the active ingredient from the adsorbent (for example, stirring for at least about 1 hour, preferably 2-10 hours, more preferably 4 hours, followed by filtration, and then washing the adsorbent with water) to obtain solution D. The step (4) can be performed in a nitrogen environment.

In one aspect, in step (5), solution D is adjusted to weakly acidic (for example, about pH 5.5-6.6, preferably about pH 6) with acid (for example, hydrochloric acid) to obtain solution E. Preferably, the step (5) can be performed under aseptic conditions. In one aspect, in step (6), the solution E is concentrated (for example, concentrated under reduced pressure, preferably concentrated by evaporation under reduced pressure, for example, at about 50°C to 70°C, preferably at about 54°C to 56°C), followed by filtration to obtain an extract containing the active ingredient. The step (6) can be performed in a nitrogen environment.

In one aspect, the patient is a mammal. In one aspect, the patient is a human, such as a middle-aged or elderly person, preferably an elderly person. The middle-aged person can be a person between 40 and 59 years old or a person between 45 and 59 years old. The elderly can be a person at or over 60 years old, at or over 65 years old, at or over 70 years old, at or over 75 years old, or at or over 80 years old.

Those skilled in the art understand that the extract of the present invention can also be obtained by inoculating other animal tissues with vaccinia virus. For example, in the present invention, an extract from an inflamed tissue inoculated with vaccinia virus can be used. The tissue may be derived from a mammalian tissue, and the mammal may comprise companion animal, laboratory animal, and livestock animal, such as rabbit, cow, horse, sheep, goat, monkey, mouse, and pig. The tissue may be skin.

### BRIEF DESCRIPTION OF THE DRAWINGS

Figure 1 shows the effects of Lepalvir on body weight changes in subacute MPTP mouse model. **p<0.01 vs. Sham; 0-7 days, n= 20 or 28; 14 days, n= 10 or 14.
Figure 2 shows the effects of Lepalvir on the total distance traveled by mouse during the open field test in subacute MPTP mouse model. ***p<0.001, **p<0.01 vs. Sham; # # # p<0.001, ## p<0.01, # p<0.05 vs. MPTP. Distance.
Figure 3 shows the effects of Lepalvir on T-Turn during the pole climbing test in subacute MPTP mouse model. **** p* <0.001 vs. Sham; *^{# # #} p* <0.001, *^{##} p* <0.01, *^{#} p* <0.05 vs. MPTP.
Figure 4 shows the effects of Lepalvir on T-TLA during the pole climbing test in subacute MPTP mouse model. **** p* <0.001 vs. Sham; *^{# # #} p* <0.001, *^{##} p* <0.01, *^{#} p* <0.05 vs. MPTP.
Figure 5 shows the effects of Lepalvir on the latency during the rotarod test in subacute MPTP mouse model. *** *p* <0.001 vs. Sham; ^{# #} *^{#} p* <0.001, *^{##} p* <0.01 vs. MPTP. Latency Time.
Figure 6 shows the effects of Lepalvir on the number of Nissl-positive neurons in the substantia nigra pars compacta of mouse in subacute MPTP mouse model. *** *p* <0.001, ***p <* 0.01 vs. Sham; *^{##}p <* 0.01, *^{#}p* < 0.05 vs. MPTP.
Figure 7 shows the effects of Lepalvir on the number of TH-positive neurons in the substantia nigra pars compacta of mouse in subacute MPTP mouse model. *** *p* <0.001 vs. Sham; *^{# # #}p* <0.001, *^{##}p* <0.01, *^{#}p <* 0.05 vs. MPTP.

### DETAILED DESCRIPTION

Unless otherwise specified, all scientific terms used herein have the same meaning as commonly understood by those of ordinary skill in the art. Exemplary methods and materials are described below, and equivalents can be used. All publications and other references mentioned herein are incorporated by reference in their entirety.

The following examples are provided to further illustrate the present invention. The following examples are not intended to limit the scope of the present invention for any reason.

### Examples

### Example 1 - Preclinical pharmacodynamic study of the therapeutic effect of Lepalvir on Parkinson's disease

### 1. Purpose of the study

To evaluate the therapeutic effect of Lepalvir on the subacute Parkinson's disease-like model induced by MPTP (1-methyl-4-phenyl-1,2,3,6-tetrahydropyridine) in mouse.

### 2. Materials and methods

### 2.1 Experimental animals

C57BL/6J mice, SPF grade, male, weighing 23-25 g. All experiments in this study were approved by the Institutional Animal Care and Use Committee of Nanjing Medical University (IACUC), ethics number: IACUC-1704001-3.

### 2.2. Drugs and reagents

Lepalvir: Extract from rabbit skin inflamed by vaccinia virus; and
Selegiline hydrochloride (Selegiline, Eldepryl).

### 2.3 Preparation of MPTP subacute Parkinson's disease-like model

The mice were weighed and recorded, and injected subcutaneously at the back of the neck at 20 mg/kg, once a day for 5 consecutive days. Subsequent experiments were performed on the 1^{st}, 3^{rd}, 7^{th} and 14^{th} days after the last administration.

### 2.4. Experimental groups and dosing

128 healthy adult mice were randomly divided into six groups according to body weight, including normal control (Sham) group (n=20), MPTP model group (n=28), low-dose Lepalvir (10 U/kg/d) treatment group (n=20), medium-dose Lepalvir (20 U/kg/d) treatment group (n=20), high-dose Lepalvir (40 U/kg/d) treatment group (n=20) and positive control drug Selegiline (0.5 mg/kg/d) treatment group (n=20). Among them, mice in the Lepalvir and Selegiline treatment groups were injected with corresponding doses intraperitoneally 1 hour after the first MPTP administration, and continued for 7 or 14 days, once a day. Mice in the MPTP model group were given an equal volume of normal saline (0.1 mL/10g). Mice in the sham group were only given an equal volume of normal saline (0.1 mL/10g).

### 2.5. Behavioral assessment

Behavioral assessment was performed on the 1^{st}, 3^{rd}, 7^{th} and 14^{th} days after the last administration of MPTP.

### a. Open field test

The open field test was used to evaluate the autonomous movement ability of mouse. The open field apparatus was made of opaque blue plastic and was 60 cm × 60 cm × 45 cm in size. During the test, each mouse was placed into the box from the central area, and the movement trajectory of each mouse within 5 minutes was recorded at the same time. The total movement distance was calculated using software (Clever Sys Inc., VA, USA). After each mouse was tested, the open field area was wiped with 75% ethanol to prevent the smell of the previous mouse from affecting the next mouse.

### b. Pole climbing test

The pole climbing test was used to evaluate the motor coordination function of mouse. The pole used was 1 cm in diameter and 50 cm in height. A wooden ball with a diameter of 1.2 cm was fixed to the top of the pole. The pole was wrapped with anti-slip tape to prevent the mouse from slipping. The mouse was placed on the top of the pole with its head facing up. When the mouse started to climb up, the timer started and the time it took for the mouse to cross the top of the pole (T- Turn) from the start of the movement and the time it took for the mouse to climb down to the bottom with four paws landing on the ground from the start of the movement (T-TLA) were recoded. Before the formal test, three consecutive trainings were required for each mouse. During the test, three consecutive measurements were required, and the shortest time was selected.

### c. Rotarod test

The rotarod test was used to evaluate the motor balance and limb coordination of mouse. The mouse was placed on the drum of the Rotarod tester, and the speed was set to increase uniformly from 5 rpm/min to 25 rpm/min within 2 minutes. When the mouse fell from the drum to the sensing area below, an infrared sensor would receive the signal and record the time the mouse moved on the drum of the instrument. Before the formal test, three consecutive trainings were required for each mouse. During the test, three consecutive measurements are required, and the average falling time was selected (latency).

### 2.6 Immunohistochemistry

On day 7 and day 14 after the last administration of MPTP respectively, half of the mice in each group were selected, anesthetized with 4% chloral hydrate, and perfused with saline + 4% PFA to obtain brain tissue. After gradient dehydration in 20% (v/v) and 30% (v/v) sucrose solutions for 3 days respectively, the mouse brain tissue was embedded in OCT glue and frozen, and the Microtome Cryostat (Leica) was used to continuously slice the tissue (20 µm), and the brain slices of the relevant regions were collected, placed in freezing solution (glycerol: 0.01M PBS = 1:1) and stored at -80°C for use.

### a. Nissl staining

Nissl bodies are a type of basophilic substance in the cytoplasm that is widely found in various neurons, and thus the number of neurons in the relevant brain regions was calculated by Nissl staining. Staining was performed using the Nissl staining kit.

### b. TH (Tyrosine hydroxylase) staining

TH mainly marks dopaminergic neurons in the substantia nigra pars compacta. After MPTP modeling, the number of TH-positive neurons in this brain region will be significantly reduced. The midbrain slices were obtained, washed once with 0.01 M PBS for 10 min, and then incubated with 3% H₂O₂ for 15 min to remove endogenous peroxidase activity. The slices were washed with 0.01 M PBS for 10 min × 3 to remove H₂O₂, then blocked with PBS containing 5% BSA and 0.3% Triton X-100 at room temperature for 1 hour, followed by incubation with mouse anti- TH primary antibody (T2928, Sigma, USA) at 4°C overnight and further incubation with secondary antibody at room temperature for 1 hour. The slices were then washed with PBS for 5 min × 3, and developed with diaminobenzidin (DAB) in the dark.

### 2.7. Cell counting

Immunohistochemical positive cells were counted using the Stereo Investigator (MBF bioscience) system and the number of Nissl body-positive cells and TH+ cells in the substantia nigra pars compacta were calculated. Cell counting was performed under a ×20 objective.

### 2.8 Data processing and analysis

All data were expressed as Mean ± SEM and statistically analyzed using the statistical analysis software GraphPad Prism 8.0. The survival rate was analyzed using the Log - rank test, and other quantitative data were analyzed using one-way ANOVA combined with Dunnett 's test for the differences between groups. *p* < 0.05 indicated that the difference was statistically significant.

### 3. Experimental Results

### 3.1. Effects of Lepalvir treatment on survival rate in subacute MPTP mouse model

Subacute administration of MPTP caused mouse to become listless, anorexic, lose weight, and develop Straub tail reaction after modeling, but no mice died in this experiment.

### 3.2. Effects of Lepalvir Treatment on body weight changes in subacute MPTP mouse model

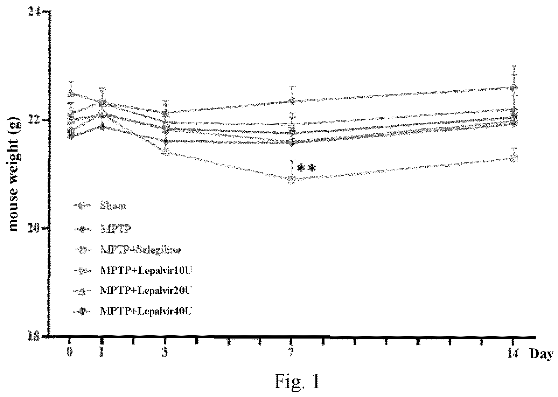

As shown in Figure 1, subacute administration of MPTP caused the mouse to eat less after modeling, and the weight of the mouse decreased slightly within 7 days. After 14 days, the food intake gradually increased and the weight gradually recovered. The experimental results showed that only the mouse in the low-dose (10U/kg/d) Lepalvir treatment group had a significant decrease in weight compared with the Sham group mouse on day 7 (p <0.01), and the treatments in other groups had no significant effect on the weight of the mouse.

### 3.3. Effects of Lepalvir on the voluntary movement ability during the open filed test in subacute MPTP mouse model

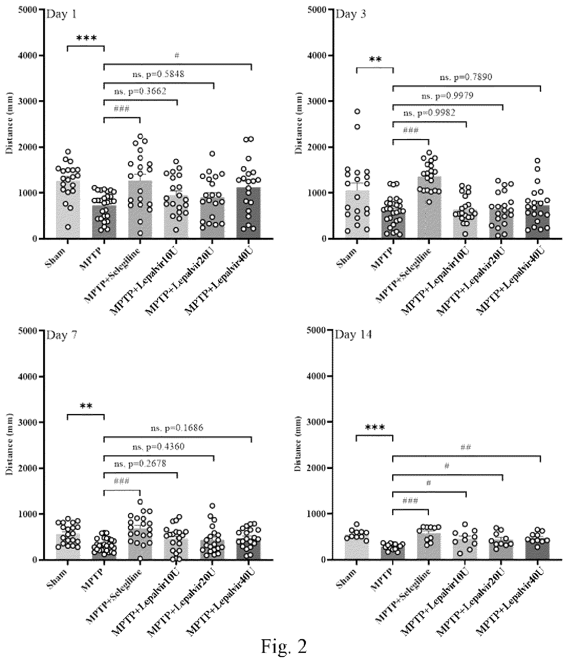

As shown in Table 1 and Figure 2, after repeated open field tests, normal mice gradually became familiar with the environment, and their locomotor activities decreased, and the total movement distance also decreased. In the subacute MPTP model, the total distance traveled by mice was reduced compared with the control group on day 1, 3, 7, and 14 after modeling, indicating a decrease in locomotor activity. The positive control drug Selegiline significantly improved the MPTP-induced reduction in total distance traveled on day 1, 3, 7, and 14, while Lepalvir had a significant improvement only on day 14. Compared with the model group, the increase in the total movement distance was 161%, 154%, and 167% for low, medium and high dose groups, respectively.

**Table 1. Effects of Lepalvir on the total distance traveled by mouse during the open filed test in subacute MPTP mouse model**

| Group | Mouse count (1-7)/14 | Total movement distance in the open field test (mm) | | | |
|---|---|---|---|---|---|
| | | 1 day | 3 days | 7 days | 14 days |
| Sham | 20/10 | 1268±87 | 1058±156 | 570±48 | 561±33 |
| MPTP | 28/14 | 727±55 *** | 604±59 ** | 328±26 ** | 284±18 *** |
| MPTP+ Selegiline | 20/10 | 1269+138 ^{###} | 1359±68 ^{###} | 684±68 ^{###} | 578±51 ^{###} |
| MPTP+Lepalvir 10U | 20/10 | 949±93 | 643±64 | 457±63 | 456±61 ^{#} |
| MPTP+Lepalvir 20U | 20/10 | 905±104 | 644±80 | 436±67 | 437±49 ^{#} |
| MPTP+Lepalvir 40U | 20/10 | 1121±129 ^{#} | 727±94 | 473±47 | 474±37 ^{##} |

| | | | | | |
|---|---|---|---|---|---|
| **** p <* 0.001, *** p <* 0.01 vs. Sham; *^{# # #}p* <0.001, *^{##}p* <0.01, *^{#}p* <0.05 *vs.* MPTP. | | | | | |

### 3.4. Effects of Lepalvir on motor coordination ability during the pole climbing test in subacute MPTP mouse model

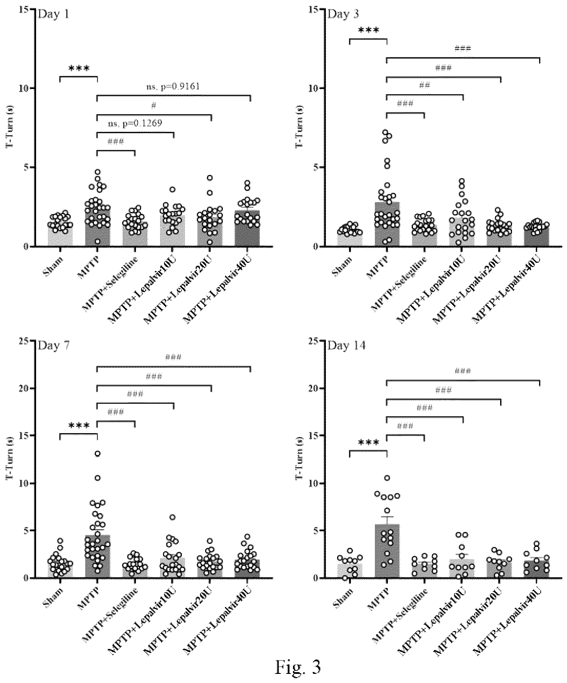

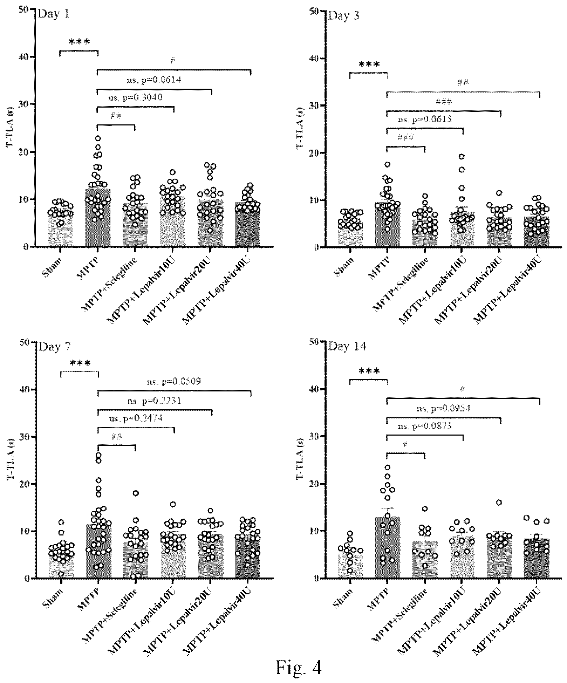

As shown in Tables 2-3 and Figures 3-4, in the MPTP subacute model, the mice showed prolonged T-Turn and T-TLA time in the pole climbing test on day 1, 3, 7, and 14 after modeling, indicating a decrease in motor coordination ability. The positive control drug Lepalvir significantly improved the MPTP-induced prolongation of T-Turn and T-TLA time on day 1, 3, 7, and 14. Lepalvir had a significant improvement effect on day 1, 3, 7, and 14. On day 14, for low, medium and high dose groups, T-Turn time was reduced to 35.1%, 28.9% and 32.6% of the model group respectively. T-TLA time also showed a decreasing trend, but there was no significant difference.

**Table 2. Effects of Lepalvir on T-Turn during the pole climbing test in subacute MPTP mouse model**

| Group | Mouse count (1-7)/14 | T-Turn (s) | | | |
|---|---|---|---|---|---|
| | | Day 1 | Day 3 | Day 7 | Day 14 |
| Sham | 20/10 | 1.53±0.08 | 1.07±0.04 | 1.59±0.20 | 1.49±0.29 |
| MPTP | 28/14 | 2.45±0.19 *** | 2.80±0.36 *** | 4.55±0.55 *** | 5.70±0.80 *** |
| MPTP+ Selegiline | 20/10 | 1.57±0.10 ^{###} | 1.35±0.09 ^{###} | 1.49±0.14^{###} | 1.49±0.20^{###} |
| MPTP+Lepalvir 10U | 20/10 | 1.96±0.14 | 1.80±0.24 ^{##} | 2.13±0.36 ^{###} | 2.00±0.51 ^{###} |
| MPTP+ Lepalvir 20U | 20/10 | 1.83±0.20 ^{#} | 1.30±0.09 ^{###} | 1.75±0.19 ^{###} | 1.65±0.27 ^{###} |
| MPTP+Lepalvir 40U | 20/10 | 2.27±0.18 | 1.26±0.05 ^{###} | 1.96±0.22 ^{###} | 1.86±0.32 ^{###} |

| | | | | | |
|---|---|---|---|---|---|
| **** p* < 0.001 vs. Sham; *^{###}p* <0.001, ^{##} *p* <0.01, *^{#}p* <0.05 vs. MPTP. | | | | | |

**Table 3. Effects of Lepalvir on T-TLA during the pole climbing test in subacute MPTP mouse model**

| Group | Mouse Count (1-7)/14 | T-TLA (s) | | | |
|---|---|---|---|---|---|
| | | Day 1 | Day 3 | Day 7 | Day 14 |
| Sham | 20/10 | 7.69±0.31 | 5.81±0.29 | 5.97±0.51 | 5.87±0.71 |
| MPTP | 28/14 | 12.22±0.87 *** | 9.58±0.59 *** | 11.47±1.15 | 13.01±1.86 |
| MPTP+ Selegiline | 20/10 | 9.23±0.65 ^{##} | 5.96±0.49 ^{###} | 7.61±0.91 ^{##} | 7.90±1.17 ^{#} |
| MPTP+Lepalvir 10U | 20/10 | 10.60±0.55 | 7.62±0.90 | 9.36±0.56 | 8.96±0.78 |
| MPTP+ Lepalvir 20U | 20/10 | 9.91±0.88 | 6.36±0.47 ^{###} | 9.30±0.63 | 9.02±0.83 |
| MPTP+Lepalvir 40U | 20/10 | 9.42±0.37 ^{#} | 6.57±0.53 ^{##} | 8.56±0.65 | 8.45±0.90^{#} |

| | | | | | |
|---|---|---|---|---|---|
| **** p* < 0.001 vs. Sham; *^{# # #}p* <0.001, ^{##}*p* <0.01, *^{#}p* <0.05 vs. MPTP. | | | | | |

### 3.5. Effects of Lepalvir on motor balance ability during the rotarod test in subacute MPTP mouse model

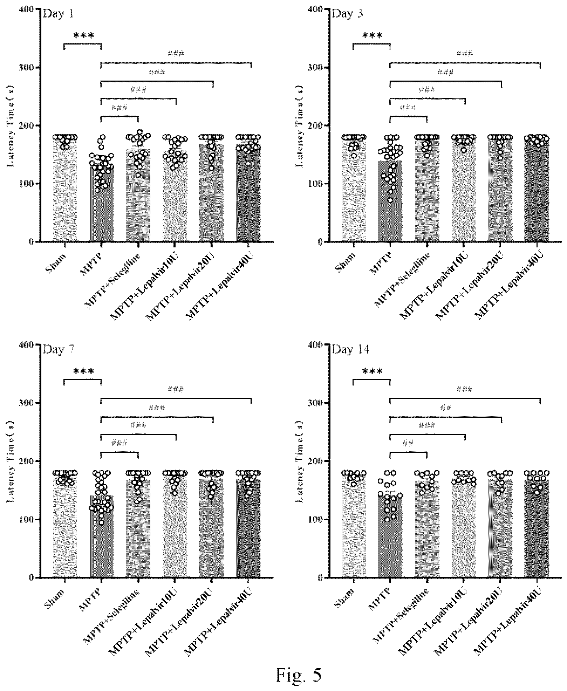

As shown in Table 4 and Figure 5, in subacute MPTP mouse model, the mice showed a shortened latency to fall in the rotarod test on day 1, 3, 7, and 14 after modeling, indicating a decrease in motor balance ability. The positive control drug Selegiline significantly improved the MPTP-induced shortening of latency on day 1, 3, 7, and 14. Lepalvir also had a significant improvement effect on day 1, 3, 7, and 14, and the latency for low, medium and high dose groups on day 14 was extended to 121.3%, 119.6% and 119.8% of the model group, respectively.

**Table 4. Effects of Lepalvir on the latency during the rotarod test in subacute MPTP mouse model**

| Group | Mouse count (1-7)/14 | Latency time (s) I | | | |
|---|---|---|---|---|---|
| | | Day 1 | Day 3 | Day 7 | Day 14 |
| Sham | 20/10 | 176.7±1.2 | 172.7±2.1 | 173.3±1.6 | 174.7±2.1 |
| MPTP | 28/14 | 129.9±4.4 *** | 139.6±5.9 *** | 141.5±4.9 *** | 141.2±6.9 ^{***} |
| MPTP+ Selegiline | 20/10 | 160.2±4.8 ^{###} | 173.1±2.2 ^{###} | 168.5±3.5 ^{###} | 166.7±4.2^{##} |
| MPTP+Lepalvir 10U | 20/10 | 157.0±4.0 ^{###} | 175.8±1.3 ^{###} | 172.9±2.3 ^{###} | 171.3±2.5 ^{###} |
| MPTP + Lepalvir 20U | 20/10 | 168.7±3.7 ^{###} | 174.9±2.2 ^{###} | 169.9±3.2 ^{###} | 168.9±4.1^{##} |
| MPTP+Lepalvir 40U | 20/10 | 168.4±2.7 ^{###} | 175.7±1.0 ^{###} | 169.3±2.9 ^{###} | 169.1±3.9 ^{###} |

| | | | | | |
|---|---|---|---|---|---|
| **** p* < 0.001 vs. Sham; *^{# # #}p* <0.001, ^{##}*p* <0.01 *vs.* MPTP. | | | | | |

### 3.6. Effects of Lepalvir on the number of Nissl-positive neurons in the substantia nigra pars compacta in subacute MPTP mouse model

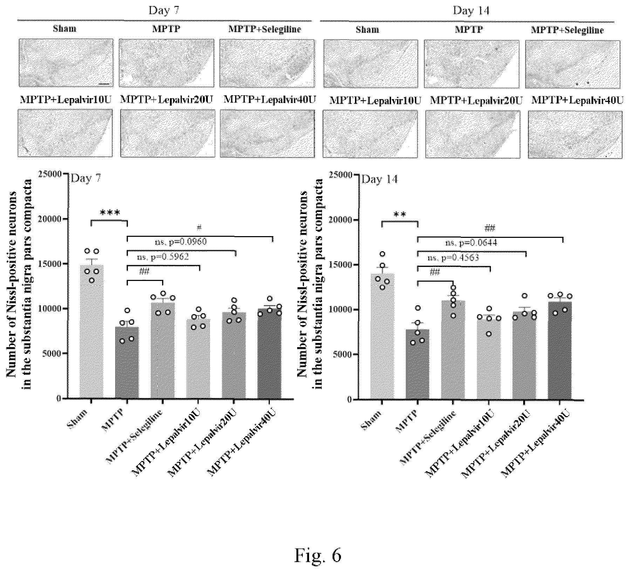

As shown in Table 5 and Figure 6, in the MPTP subacute model, the number of Nissl-stained positive neurons in the substantia nigra pars compacta decreased in mice on day 7 and 14 after modeling, indicating the death of neurons in this brain region. The positive control drug Selegiline significantly improved the MPTP-induced decrease in the number of Nissl-stained positive neurons in the substantia nigra pars compacta on day 7 and 14. The high-dose group of Lepalvir had a significant improvement on day 7 and 14, and the number of Nissl-stained positive neurons increased to 126.3 and 139.6% respectively compared with the model group.

**Table 5. Effects of Lepalvir on the number of Nissl-positive neurons in the substantia nigra pars compacta in subacute MPTP mouse model**

| Group | Mouse count 7/ 14 | Number of Nissl-positive neurons in the substantia nigra pars compacta | |
|---|---|---|---|
| | | Day 7 | Day 14 |
| Sham | 515 | 14875±661 | 14058±677 |
| MPTP | 515 | 7985±637 *** | 7814±710 ** |
| MPTP+ Selegiline | 515 | 10688±458 ^{##} | 11024±539 ^{##} |
| MPTP+Lepalvir 10U | 515 | 8874±378 | 8974±456 |
| MPTP+ Lepalvir 20U | 515 | 9660±436 | 9841±448 |
| MPTP+Lepalvir 40U | 515 | 10083±314 ^{#} | 10905±443^{##} |

| | | | |
|---|---|---|---|
| **** p* <0.001, *** p* < 0.01 vs. Sham; ^{##} *p* < 0.01, *^{#}p* < 0.05 vs. MPTP | | | |

### 3.7. Effects of Lepalvir on the number of TH-positive neurons in the substantia nigra pars compacta in subacute MPTP mouse model

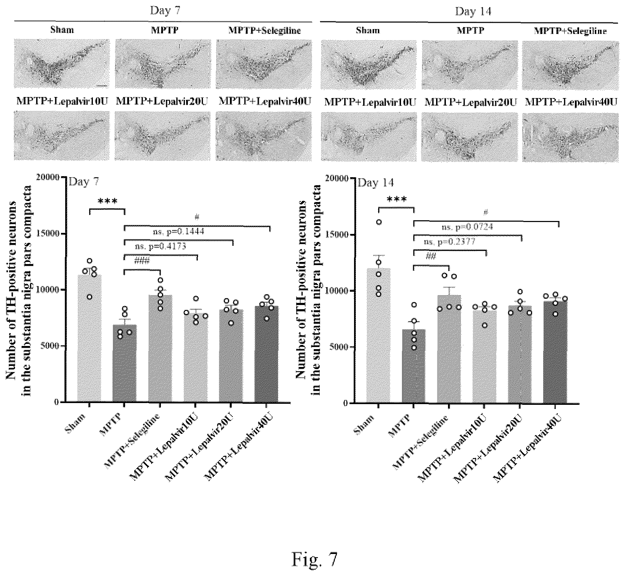

As shown in Table 6 and Figure 7, in the MPTP subacute model, the number of TH-positive neurons in the substantia nigra pars compacta of mouse decreased on day 7 and 14 after modeling, indicating the death of dopaminergic neurons in this brain region. The positive control drug Selegiline significantly improved the MPTP-induced decrease in the number of TH-positive neurons in the substantia nigra pars compacta on day 7 and 14. Only the high-dose group of Lepalvir had a significant improvement on day 7 and 14, and the low and medium-dose groups showed a trend of improvement, but there was no significant difference. On day 7, the number of TH-positive neurons in the low, medium and high-dose groups increased to 115.2%, 120.3% and 125.3% of the model group, respectively, and on day 14, the number of TH-positive neurons in the low, medium and high-dose groups increased to 125.7%, 132.7% and 138.7% of the model group, respectively.

**Table 6. Effects of Lepalvir on the number of TH-positive neurons in the substantia nigra pars compacta in subacute MPTP model**

| Group | Mouse count 7/ 14 | Number of TH-positive neurons in the substantia nigra pars compacta | |
|---|---|---|---|
| | | Day 7 | Day 14 |
| Sham | 515 | 11365±539 | 12025±1138 |
| MPTP | 515 | 6895±494 *** | 6587±672 *** |
| MPTP+ Selegiline | 515 | 9578±441 | 9670±692^{##} |
| MPTP+Lepalvir 10U | 515 | 7945±358 | 8281±341 |
| MPTP+ Lepalvir 20U | 515 | 8294±352 | 8741±356 |
| MPTP+Lepalvir 40U | 515 | 8641±327^{#} | 9139+314 ^{#} |

| | | | |
|---|---|---|---|
| **** p* < 0.001 vs. Sham; *^{# # #}p* <0.001, ^{##}*p* <0.01, *^{#}p* < 0.05 *vs.* MPTP. | | | |

### 4. Conclusion

Lepalvir treatment for 7 and 14 days can improve the neurobehavioral disorders of mouse in the subacute MPTP model. The high-dose group (40 U/kg/d) had the most significant effect on day 14. Compared with the model group, the high-dose group can increase the total movement distance in the open field test to 167%, shorten the T-Turn time and the T-TLA time during the climbing pole test to 32.6% and 65.0%, and increase the latency during the rotarod test to 119.8%. At the same time, the number of Nissl-stained positive neurons in the high-dose Lepalvir group increased to 126.3% (day 7) and 139.6% (day 14) compared with the model group, and the number of TH-positive neurons in the substantia nigra pars compacta increased to 125.3% (day 7) and 138.7% (day 14) compared with the model group. These results show that Lepalvir has the effect of alleviating dopaminergic neuron damage in the subacute MPTP model. Continuous administration of Lepalvir for 7 and 14 days has an alleviating effect on Parkinson's disease-like changes in the mouse.

## Claims

1. Use of extract from rabbit skin inflamed by vaccinia virus in the preparation of a medicament for preventing or treating Parkinson's disease in a patient.

2. The use of claim 1, wherein the medicament is used to restore neurological function of the brain or alleviate damage to neurological function of the brain in a patient suffering from Parkinson's disease.

3. Use of extract from rabbit skin inflamed by vaccinia virus in the preparation of a medicament for alleviating or reducing neuronal degeneration, damage or death in the brain of a patient suffering from Parkinson's disease.

4. The use according to any one of claims 1 to 3, wherein the patient suffers from parkinsonism caused by Parkinson's disease.

5. The use according to any one of claims 1 to 4, wherein the patient has motor symptoms such as tremor, limb rigidity, bradykinesia or postural instability ; neuropsychiatric disorder such as speech disorder, cognitive disorder, emotional disorder or thought disorder; sensory disorder or sleep disorder; or autonomic dysfunction such as excessive oil secretion, hyperhidrosis or urinary incontinence.

6. The use according to any one of claims 1 to 5, wherein Parkinson's disease is caused by aging, genetic factors, environmental toxins or pathogen infection.

7. The use according to any one of claims 1 to 6, wherein the patient is **characterized by** a decrease, damage or death of neurons in the substantia nigra pars compacta of the brain.

8. The use according to any one of claims 1 to 7, wherein the extract from rabbit skin inflamed by vaccinia virus is Lepalvir.

9. The use according to any one of claims 1 to 8, wherein the extract from rabbit skin inflamed by vaccinia virus is formulated into an oral preparation or an injection, preferably an intramuscular injection or an intravenous injection.

10. The use according to any one of claims 1 to 9, wherein the patient is a human, preferably an elderly person.

11. The use according to any one of claims 1 to 10, wherein the extract from rabbit skin inflamed by vaccinia virus is administered to the patient in an amount of 0.05 U/kg to 50 U/kg, preferably 0.1 U/kg to 10 U/kg, more preferably 0.5 U/kg to 5 U/kg.

12. The use according to any one of claims 1 to 11, wherein the medicament comprises 3 U to 3000 U, preferably 6 U to 600 U, more preferably 30 U to 300 U of the extract from rabbit skin inflamed by vaccinia virus.
